(19) Europäisches Patentamt / European Patent Office / Office européen des brevets

(11) **EP 3 046 632 B1**

(12) **EUROPEAN PATENT SPECIFICATION**

(45) Date of publication and mention
of the grant of the patent:
**13.06.2018 Bulletin 2018/24**

(21) Application number: **14777917.7**

(22) Date of filing: **18.09.2014**

(51) Int Cl.:
*A61Q 11/00* (2006.01)          *A61K 8/19* (2006.01)
*A61K 8/42* (2006.01)          *A61K 33/06* (2006.01)
*A61K 33/02* (2006.01)          *A61K 33/00* (2006.01)
*A61K 33/40* (2006.01)          *A61K 31/17* (2006.01)

(86) International application number:
**PCT/NL2014/050637**

(87) International publication number:
**WO 2015/041523 (26.03.2015 Gazette 2015/12)**

(54) **ORAL HYGIENE COMPOSITIONS**

MUNDPFLEGEZUSAMMENSETZUNGEN

COMPOSITIONS D'HYGIÈNE BUCCALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.09.2013 EP 13184923**

(43) Date of publication of application:
**27.07.2016 Bulletin 2016/30**

(73) Proprietor: **Glymur B.V.**
**3844 NC Harderwijk (NL)**

(72) Inventors:
• **CRIELAARD, Willem**
**NL-7314 PC Apeldoorn (NL)**
• **BRUGMAN, Martijn**
**NL-3844 NC Harderwijk (NL)**
• **BRUS, Ronald Hendrik Peter**
**NL-2253 AD Voorschoten (NL)**
• **KLEIN, Bart**
**NL-2626 NC Delft (NL)**

(74) Representative: **Nederlandsch Octrooibureau**
**P.O. Box 29720**
**2502 LS The Hague (NL)**

(56) References cited:
WO-A1-96/19228          WO-A1-2004/108173
DE-A1-102007 050 767          US-A- 6 036 944

• M. GILCHRIST ET AL: "Inorganic nitrate and nitrite and control of blood pressure", CARDIOVASCULAR RESEARCH, vol. 89, no. 3, 29 September 2010 (2010-09-29), pages 492-498, XP055103418, ISSN: 0008-6363, DOI: 10.1093/cvr/cvq309
• JUSTIN JOHNSTON DOEL ET AL: "Evaluation of bacterial nitrate reduction in the human oral cavity", EUROPEAN JOURNAL OF ORAL SCIENCES, vol. 113, no. 1, 1 February 2005 (2005-02-01), pages 14-19, XP055103393, ISSN: 0909-8836, DOI: 10.1111/j.1600-0722.2004.00184.x
• Anonymous: "Geurverwijdering", A Blue World Company , 4 March 2013 (2013-03-04), pages 1-2, XP002720777, Retrieved from the Internet: URL:https://web.archive.org/web/2013030402 1648/http://ablueworldcompany.com/geurverw ijdering/ [retrieved on 2014-02-19]
• ALLAKER R P ET AL: "Topographic distribution of bacteria associated with oral malodour on the tongue", ARCHIVES OF ORAL BIOLOGY, vol. 53, 1 April 2008 (2008-04-01), pages S8-S12, XP022638913, PERGAMON PRESS, OXFORD, GB ISSN: 0003-9969, DOI: 10.1016/S0003-9969(08)70003-7 [retrieved on 2008-04-01]

**Description**

**FIELD OF THE INVENTION**

The invention relates to compositions and their use for improving the health of the oral cavity in animals and humans. More in particular, the invention relates to such compositions and their use to treat and/or prevent halitosis.

**BACKGROUND OF THE INVENTION**

Halitosis (bad breath) constitutes an underestimated problem. The problem of bad breath not only hampers an individual in its social life, it is also a sign of poor oral hygiene, with potentially more severe health implications.

Hydrogen sulphide ($H_2S$), methyl mercaptan ($CH_3SH$), and dimethyl sulphide ($CH_3SCH_3$), collectively referred to as volatile sulphur compounds (VSC) are the principal malodourant components in bad breath. VSC originate from the anaerobic degradation of sulphur-containing amino acids by bacteria in the oral cavity. As substrates for odor production, the bacteria mainly utilize the amino acids methionine and cysteine present in protein left behind in the oral cavity following a protein containing meal. These amino acids contain sulfur and are metabolized by the bacteria to yield volatile sulfur compounds. These substances have an unpleasant odor, even in extremely low concentrations. It has been shown that anaerobic Gram-negative bacteria are primarily responsible for this degradation.

The magnitude of the problem of halitosis may be appreciated from the approximate size of the market for mouth wash products, which is roughly a billion US Dollars per annum in the United States of America alone. Unfortunately, most products such as mouth washes and rinses that are commercially available do not work, or at best temporarily. Moreover, many products may be considered to worsen a person's oral hygiene and/or health status since most products aim at killing the mouth flora in order to reduce the catabolic processes that cause VSC production. However, in the process of killing the anaerobic Gram-negative bacteria responsible for the production of VCS, traditional mouth washes destroy the entire mouth flora, including bacteria that are seminal for good oral cavity health. In other words, the mouth waters of the prior art do nothing to restore a healthy bacterial population in the oral cavity. Quite on the contrary, traditional mouth waters add to the problem of a disbalance in the bacterial population.

The most common strategy used in the treatment of halitosis is killing the bacterial population inside the oral cavity using mouth wash compositions that comprise antibacterial compounds.

For example WO0174322A1 discloses oral hygiene compositions comprising the cationic antibacterial agent, chlorhexidine, for combating halitosis.

Well known commercially available mouth hygiene products are Peridex (Procter & Gamble) containing the bactericide chlorhexidine gluconate and Cool Mint Listerine® (Johnson & Johnson). Listerine® is the trademark for an antiseptic solution containing boric acid, benzoic acid, thymol, and essential oils of Eucalyptus, Gaultheria, sorbitol solution, 21.6 percent alcohol, Poloxamer 407, benzoic acid, flavoring, sodium saccharine, sodium citrate, citric acid and colorant.

US2008299051A discloses pharmaceutical compositions for the treatment of periodontal disorders, including halitosis. The compositions consist of a mixture of hydrogen peroxide, eugenol, permonochlorophenol, camphor, maltitol, coloring matter and appetizing substance water.

US4525342 discloses compositions comprising highly hydrophobic compounds, such as hydrocarbons, vegetable and/or mineral oils. By binding to the outer surface of bacteria, these are effectively removed from the oral cavity and from the surface of teeth. The compositions are stated to be effective in the removal of odor-forming constituents and thus for the alleviation of or removal of the effects of halitosis.

Many mouthwash formulations described in the patent literature for use in oral hygiene utilize quaternary ammonium compounds or benzalkonium chloride as a bactericide. Illustrative of these are U.S. Pat. Nos. 4,110,429, 5,145,664, 5,362,737 and 5,374,418.

US2004067204A discloses the use of antioxidants or antioxidant-containing compositions for treating halitosis, such as vitamins, coenzyme Q,amino acids, imidazoles, peptides, fatty acids, metal compounds and derivatives of these compounds (for example salts, esters, ethers, sugars, nucleotides, nucleosides).

Among the most common mouth rinses are those that comprise quaternary amines (e.g., combinations of ethanol and domiphen bromide and/or cetylpyridinium chloride) or mixtures of orally acceptable surface-active agents or surfactants. Several mouth rinses that have been marketed for the reduction of plaque and gingivitis generally rely on cationic agents such as chlorhexidine digluconate, metallic fluoride salts such as stannous fluoride, antimicrobial essential oils (e.g., thymol, eucalyptol, ethanol, menthol and methyl salicylate) and/orwater-insoluble phenolic agents such as triclosan.

Cationic antimicrobial materials such as chlorhexidine, benzethonium chloride, and cetyl pyridinium chloride have been investigated as antimicrobial agents in compositions for the control of gingivitis and/or halitosis. The antimicrobial activity of these compounds is most probably connected to the positive charge of these molecules. Because of their positive charge these compounds are attracted to negatively-charged moieties on the cell membrane or wall of the microorganism, which facilitates attachment to the surface of the microorganism. The attachment and subsequent interaction with the cell surface disrupts the cell membrane structure causing leakage of the intracellular fluids, eventually killing the microorganism. US2005239903A discloses compositions for the treatment of halitosis using phenyl substituted phenol compounds exhibiting antimicrobial activity, antimicrobial compositions containing phenyl substituted phenol compounds, and methods of using such compositions. WO11094872A1 discloses liquid oral care compositions, particularly in the form of a mouth spray or gurgling rinse, comprising non-ionic surfactants, flavoring oils and precursors of ClO2. CN102218021A discloses a toothpaste for treating halitosis, which comprises by weight percentage: 20 to 30 percent of calcium carbonate, 20 to 30 percent of silicon dioxide, 10 to 15 percent of sorbierite, 10 to 15 percent of glycerol, 1.0 to 2.0 percent of naringin, 1.0 to 2.0 percent of zinc citrate, 0.1 to 0.2 percent of cineole, 1.5 to 2.0 percent of laurinol sodium sulfate, 0.5 to 1.0 percent of hydroxymethyl sodium cellulosate, 1.0 to 2.5 percent of sodium saccharin, 1.0 to 1.5 percent of essence, dissolved in water. DE102007050767A1 discloses anti-bacterial and bleaching oral care and treatment compositions, also against halitosis, which rely on peroxides like UHP for treating halitosis.

It is furthermore known that water-soluble salts of certain metals, such as divalent cations of zinc, copper and tin, can inhibit oral malodor. The metals mentioned above have high affinity for sulfur and eliminate the VSC by forming insoluble sulfur compounds, which inhibits further formation of the odoriferous gases in the oral cavity.

Water soluble, cationic, antibacterial agents such as the bis-biguanides and the quaternary ammonium compounds are reportedly able to inhibit oral malodor when used as mouth rinses. Japanese Patent Application JP 1996/356310 (Publication JP 98182384 A2) discloses a composition which can be used for the prevention of bad breath, comprising triclosan, sodium bicarbonate, menthol and other fragrances.

Japanese Patent Application JP 1988/317621 (Publication JP 90164816 A2) relates to an aqueous composition containing ferrous sulfate and sodium bicarbonate for treating halitosis. Japanese Patent Application JP 1985/39538 (Publication JP 86197510 A2) relates to a composition in the form of a toothpaste, tooth powder, mouthwash, gingival massage cream or local liquid or a paste paint, containing nitroimidazole and extracts from leaves of camellia plant such as tea tree or camellia or sodium copper-chlorophyllin. The composition is used for the prevention of periodontal diseases and reportedly inhibits gram negative, anaerobic microorganisms from forming volatile sulfur compounds in the oral cavity.

WO 99/56714 relates to a bactericide for detergents which comprises an inorganic support having antifungal metal ions supported thereon, and a denture detergent containing the bactericide. The bactericide is specifically intended for dentures and it is stated that it suppresses bad breath and denture-specific stomatitis.

The article "Effects of new flavonoid gums eliminating bad breath", Shokuhin Kogyo (SKGYAQ, 05598990); 1995; Vol.38 (4); pp. 70-8, relates to a chewing gum containing green tea flavonoids, chlorophyll copper complex and Hovenia dulcis. It is used for eliminating bad breath.

GB2381449A discloses oral hygiene compositions suitable for the treatment of halitosis and periodontal disease comprising a nitro-containing antibacterial compound, such as the nitrofurans nitrofurantoin and nitrofurazone and the 2-, 4- or 5-nitroimidazoles benzimidazole, nimarozole, tinidazole and metronidazole. The compositions may be in various forms, including as a dentifrice, a mouthwash, a gel or a solid matrix.

Another strategy in combating halitosis is to add compounds that aid in complexation and/or degradion of VCS.

It is known that aqueous solutions of zinc salts used as mouth rinses reduce and inhibit VSC formation in the oral cavity. It is assumed that zinc ions form stable mercaptides with the substrate, with precursors of VSC or with the VSC directly, since zinc has an affinity for sulphur and oxidizes sulphhydryl groups. It has for example been established that zinc-containing chewing gum has an effect on VSCs in the oral cavity (S. M. Waler: The effect of zinc-containing chewing gum on volatile sulfur-containing compounds in the oral cavity; Acta Odontol. Scand. 55 (1997); p. 198-200).

Canadian patent application no. 2,154,860 relates to an oral care product which contains alkali metal pyrophosphate and a water-soluble zinc polyamine complex capable of releasing zinc ions in an environment such as the oral cavity.

US2012148506A discloses a poly-antiseptic antimicrobial pharmaceutical composition for oral use, for the hygiene and treatment of oral diseases of bacterial etiology, including halitosis, consisting of a mixture of hydrogen peroxide, eugenol, natural camphor, zinc sulphate, sodium fluoride, xylitol, cetylpyridinium chloride and excipients.

KR20030072766A discloses an oral hygiene composition containing chlorite ions encapsulated in a liposome and zinc ion. The composition can extend the remaining time of chlorite ions which can chemically decompose volatile sulfur compounds within the oral cavity.

Yet another strategy in treating halitosis concerns the provisioning of mutant bacteria, as a probiotic.

For example, US2006246015A discloses compositions comprising one or more isolated LDH-deficient mutant streptococcus strains and one or more isolated S. oralis strains and/or one or more isolated S. uberis strains.

Where the prior art predominantly advocates the use of antibacterials as a way of treating halitosis, the present inventors consider such use of antibacterials to be a major disadvantage of the mouth hygiene compositions of the prior art. The antibacterials used in the mouth wash compositions of the prior art are indiscriminate in that they kill, or at least decimate, the entire mouth flora, including the bacterial species that are important for oral cavity health.

It is an object of the present invention to provide compositions for treating halitosis without the disadvantages of the compositions of the prior art.

## SUMMARY OF THE INVENTION

The invention in one aspect provides an undiluted oral hygiene composition comprising 20 - 80 % nitrates of which at least half the amount of nitrates consists of calcium nitrate, the remainder of the nitrates being selected from any pharmaceutically acceptable cation salt thereof, 5 - 30% UHP, 0 - 20% urea, and water adding up to 100%.

Preferably, said undiluted oral hygiene composition comprises 30 - 75% nitrates, more preferably 40 - 70% nitrates, still more preferably 50 - 65% nitrates. Said undiluted oral hygiene composition preferably comprises as other nitrates at least ammonium nitrate and optionally potassium nitrate.

In a further aspect, the invention provides a diluted oral hygiene composition obtainable by diluting an undiluted oral hygiene composition according to the invention with water, or some other diluents compatible with it, wherein the dilution factor is selected from the group consisting of a between 1:1 and 1:10, between 1:10 and 1:50, between 1:50 and 1:00 and between 1:100 and 1:1000.

In a further aspect, the invention provides an oral hygiene composition according to the above, for the use in the treatment and/or prevention of halitosis in a human or animal.

In a further aspect of the invention, a composition is provided that is rich in nitrates, according to claim 1 for use in the treatment and/or prevention of halitosis in a human or animal. Said composition is one, wherein said nitrates make up 20 - 80 % of the composition, wherein at least half the amount of nitrates consists of calcium nitrate, the remainder of the nitrates being selected from any ammonium nitrate, potassium nitrate or any pharmaceutically acceptable cation salt thereof. Said composition further comprises UHP and optionally urea. Said composition is preferably for oral administration to a subject in need thereof, but other ways of administration are not excluded.

## DESCRIPTION OF THE FIGURES

Fig. 1 shows the effect of various concentrations of BM500, Listerine and CB12, present in the growth medium, on the viability of 72 hour old microcosm biofilms

Fig. 2 shows the effect of 2000 and 1000 ppm of BM500, 500 times diluted CB12, present in the growth medium, on the viability of 72 hour old microcosm biofilms of two different saliva donors.

Fig.3 shows the effect of the addition of either 1000 and 2000 ppm BM500 to the growth medium on the production of Volatile Sulphur Components of 72 hours microcosm biofilms.

Hydrogen Sulphide ($H_2S$), Methylmercaptane ($CH_3SH$) and dimethyl sulphide ($CH_3)CS$ concentrations are shown as part per billion (ppb).

Figure 4a and 4b show the effect of the addition of either 1000 or 2000 ppm BM500 respectively to the growth medium on the microbial diversity of 72 hours microcosm biofilms derived from 4 separate saliva's.

Figure 5 shows a flowchart of the study design and overview of the procedures that the subjects will undergo in the course of the study.

## DETAILED DESCRIPTION OF THE INVENTION

The present invention provides mouth hygiene compositions that reduce VSC by changing the growth conditions in the oral cavity in favor of anaerobic respiration. Anaerobic respiration is a form of respiration that involves electron acceptors other than oxygen. Examples of such alternative electron acceptors are sulfates, nitrates, sulfur and fumarate. In accordance with the invention, a shift towards anaerobic respirational growth of the microflora in the oral cavity, is brought about using an oral hygiene composition that is rich in nitrates. Such compositions considerably reduce the production of VSC compounds, especially hydrogen sulfide. Moreover, it has been established that by using oral hygiene compositions according to the invention, the overall diversity of the mouth flora is not diminished, but rather shifted from an halitotic (strictly anaerobically growing, hydrogen sulfide producing) bacterial population to a bacterial population capable of anaerobic respirational growth. To the best of the inventors' knowledge, skewing the oral cavity flora from strict anaerobic to anaerobic respirational growth by offering a composition that is compatible with and suitable for use as a oral hygiene composition in animals and humans constitutes a new approach towards promoting oral cavity health in general, and in particular to the treatment and or prevention of halitosis in mammals.

US2009324547A discloses an oral composition comprising a combination of probiotic bacteria selected from the group comprising Streptococcus, Eubacterium, Neisseria, Veillonella and pH-rising and/or buffering components, which re-establishes an oral microflora associated with good oral health in subjects with a disturbed oral microflora. The invention also discloses the use of the oral composition in combination with other oral health promoting products to help subjects suffering from disorders such as oral dryness, caries, halitosis, inflamed oral mucous membranes or oral fungal infections to restore good oral health. Although the object of the inventors in US2009324547 may be similar, the approach chosen is an entirely different one.

In accordance with the present invention the mouth flora composition is not altered by adding bacteria from the outside, but by changing the conditions that determine the composition of the mouth flora. The invention does so, by stimulating anaerobic respirational growth by adding a composition that comprises a number of components that favor the growth of bacteria capable of anaerobic respirational growth, at the expense of strict anaerobic bacteria that are responsible for the formation of VSC.

More in particular, the invention provides mouth hygiene compositions rich in nitrates, such as ammonium nitrate and especially calcium nitrate. Sodium nitrate, potassium nitrate, and magnesium nitrate may be present, but they are not essential. Preferred nitrates in compositions according to the invention are calcium nitrate and ammonium nitrate. In addition, the mouth hygiene composition according to the invention provides hydrogen peroxide - urea, also known as urea peroxide, percarbamide, carbamide peroxide or simply as UHP (hereinafter UHP), and optionally creatinine. Preferred according to the invention are compositions rich in calcium nitrate and UHP.

Without wishing to be bound by theory, the nitrate ions are believed to be important components in the oxidation pathway of aerobic and facultative bacteria in an anaerobic environment.

Each of the anions in the nitrate salt, primarily $Ca^{++}$ and $NH_4^+$ (and optionally $K^+$) serve their own purpose and are believed to be important. Although the presence of all three is preferred, the presence of all three is not essential.

The calcium ion, when added to the halitotic oral cavity with its typically higher sulfide composition, will precipitate out calcium sulfate. This lowers the amount of sulfate ions available to anaerobic bacteria as their oxidant source. Combined with the addition of the nutrient source for oxidation, this compound plays an important role in ensuring that the bacteria capable of anaerobic respirational growth out-compete the strictly anaerobic bacteria. Calcium is also a source of nutrition for cell development in bacteria. In addition, the precipitation of sulfate formed from sulfide will directly contribute to a reduction of VSC and thereby directly reduce the symptoms of halitosis.

Potassium is an important building block for cell nutrition and growth. The potassium ion is a preferred component of the composition according to the invention, as it provides for selective growth promotion of bacteria capable of anaerobic respirational growth.

The ammonium ion functions as an electron donor in the respiratory path of anaerobic respiring bacteria. It is therefore believed to contribute to the selective growth promotion of such bacteria in the oral cavity.

Micro-organisms such as bacteria require a carbon source for protein synthesis. The composition according to the invention provides such a carbon source in addition to nutrients for the anaerobically respiring bacteria that use nitrate as their respiratory oxidant. Hence the presence of UHP and/or urea effectively and selectively stimulates the growth of such bacteria in the oral cavity. Urea in addition to UHP is preferred but not essential.

The composition according to the invention in undiluted form comprises between 20 and 80 percent calcium nitrate, preferably between 30 and 75%, most preferably between 40 and 70%, still more preferably between 50 and 65%. The composition according to the invention comprises between 5 and 30 % UHP, more preferably between 10 and 25%, still more preferable between 15 and 20% UHP. In addition the composition according to the invention comprises between 0 and 20% urea, more preferably between 2 and 15%, still more preferably between 5 and 10% urea. Preferably, a fraction of totality of the nitrates salts is in the form of ammonium nitrate and/or potassium, preferably both. The presence of sodium nitrate is entirely optional. The components should make up 100% together with a minimum of water. Calcium nitrate is extremely soluble in water. In fact the composition according to the invention is difficult to obtain in dry form as it tends to attract water and become a solution more or less spontaneously.

Most preferred according to the invention is a composition comprising in undiluted form between 60 and 65% Calcium nitrate, between 15 and 20% UHP and between 5 and 10% urea, the remainder being water adding up to 100%.

Oral hygiene compositions according to the invention may be sold and used in undiluted form. Alternatively, the oral hygiene composition may be sold and used in diluted form. Useful dilutions are for example dilutions between 1:1 and 1:1000, preferably 1:100, still more preferably 1:50, even more preferably 1:10. If sold in concentrated form, the end user may dilute the oral hygiene composition by adding water or some other fluid that is compatible with the oral hygiene composition according to the invention or its intended use.

Oral hygiene compositions of the present invention maybe provided in any of the presentations normally used for such products, for instance, dentifrices including toothpastes and toothpowders, abrasive and non-abrasive gels, mouthwashes, gargles, irrigating solutions, mouth sprays and presentations for sucking or chewing by the user such as gums, pastilles, lozenges and wafers which can rapidly dissolve in the mouth. Components for the orally acceptable carrier or excipient will be selected according to the particular type of presentation involved.

The pH of the composition is preferably compatible with oral tissues, typically between 4 and 9, more preferably between 5 and 8. The composition can optionally include non-active additives to enhance the appearance or taste of the composition, for example, and coloring and/or, flavoring agents as are known in the art. Non-limiting examples of flavoring agents include the mint-flavorings such as oil of spearmint, oil of peppermint and oil of wintergreen, and other oils including citrus, clove, eucalyptus, etc. Colorants may be chosen from those approved by the FDA, such as Blue Nos. 1 and 2. Green No. 6, Red Nos. 3 and 40, and Yellow Nos. 5 and 6. Non-fermentable sugars or sugar substitutes may also be added where a sweetened vehicle is desired. These include sugar alcohols, sorbitol, xylitol, maltitol, saccharines, aspartame, sucaryl or the like. Flavorants and sweeteners are used in small amounts, e.g., up to 0.25 weight percent, preferably up to 0.05 weight percent.

Known anti-stain additives e.g., in an amountof 0.01 to 0.1 weight percent, may also be added, such as phosphorous-containing and organo-phosphorous-containing compounds. But for staining, which may occur, it is preferred to utilize the composition of the invention both as a mouth rinse and incorporated into a toothpaste, for example as a gel component. In a preferred treatment method to avoid staining, both a mouth rinse and a tooth brushing with the composition of the invention are undertaken in the morning, while in the evening, the mouth rinse alone is used. However, the composition can always be used in mouthwash form alone.

Whenever reference is made herein to oral hygiene compositions, these will be understood to include at least one component not typically present in products rich in nitrates and UHP and used or promoted for waste water treatment, agricultural or industrial use, or similar non pharmaceutical or cosmetic treatments. Such components may be selected from colorants, flavorants, fragrances, sweeteners and texture improvers, such as gels (carrageenans) and the like.

The compositions according to the invention stimulate bacteria to use electron acceptors other than oxygen, such as nitrates, sulphates, sulfur or fumarate. This process is referred to in the literature as anaerobic respiration. Preferably, the compositions according to the invention are rich in nitrates, stimulating bacteria to use nitrates as electron acceptor under anaerobic conditions. Hence, the compositions according to the invention, when administered to a subject in need thereof, preferably orally, stimulate nitrate reduction. The concomitant production of nitrite from nitrate, is believed to have several medical benefits associated with it. For example, nitrite is considered to be required for the production of NO, which is regulator of blood pressure. These and other medical benefits are considered to be part of the invention. Hence, the compositions according to the invention are also useful as a medicament, not just for treating halitosis, but for treating other disorders such as high blood pressure and the like.

Treatment of halitosis and other disorders associated with the oral cavity, such as gingivitis, caries and the like, are all encompassed by the invention, regardless whether such treatment is on medical indication or by way of cosmetic treatment.

The following example is used to illustrate the invention and is by no means intended to limit the scope of the invention.

## EXAMPLES

### EXAMPLE 1; Effect of compositions according to the invention on the bacterial growth, bacterial diversity and the formation of VSC in the oral cavity of humans.

To study the effect of compositions according to the invention on the bacterial growth, bacterial diversity and the formation of VSC in the oral cavity of humans, we took dilutions of BM500™ a commercially available product (A Blue World Company B.V.) rich in nitrates and UHP and tested them in a biofilm model developed at ACTA (Amsterdam).

### Effect of BM500™ on bacterial viability

The ACTA biofilm model system (Exterkate et al, Caries Research 2010 Vol 44 (4) p372-379. 2010) was used as a basis for these experiments. Briefly, sterile microscope-glass-slides mounted on a stainless steel lid, were suspended in a 24-well microtiter plate containing a saliva derived inoculum. Stimulated saliva was diluted 50-fold in the inoculation medium consisting of 2.5 g/l mucin, 2.0 g/l Bacto peptone, 2.0 g/l Trypticase peptone, 1.0 g/l yeast extract, 0.35 g/l NaCl, 0.2 g/l KCl, 0.2 g/l $CaCl_2$, 1 mg/l hemin, and 0.2 mg/l vitamin K1 according to McBain et al Journal of Applied Microbiology (2005) 98:p624-634, at pH 7.0. After 8h incubation at 37°C under anaerobic condition, the glass-slides were transferred to a 24-well plate containing fresh medium without inoculum and incubated at the same conditions for another 16 h. The slides were subsequently moved to a 24-well plate containing fresh medium with the addition of a tenfold diluted active compound to final concentrations of either 2000, 1000, 100, 25, 10 and 1 ppm BM500, 10% Listerine, 10% and 4% and 0.2% CB12 and MilliQ water as a negative control. The glass slides were incubated for an additional 48 hrs under anaerobic conditions at 37°C with media refreshments, containing the active compounds, at 8, 24 and 32 hours.

The 72 hrs microcosm biofilms were harvested, by removing the glass slides from the stainless steel lids, and transferred to sterile tubes containing cysteine peptone water. The glass sides were subsequently sonicated, serially diluted and plated onto tryptic soy blood agar, followed by an incubation for 72 hours at 37°C under anaerobic condition. The bacterial viability was determined by counting the amount of colony forming units (CFU) per plate and correcting them for the proper dilutions.

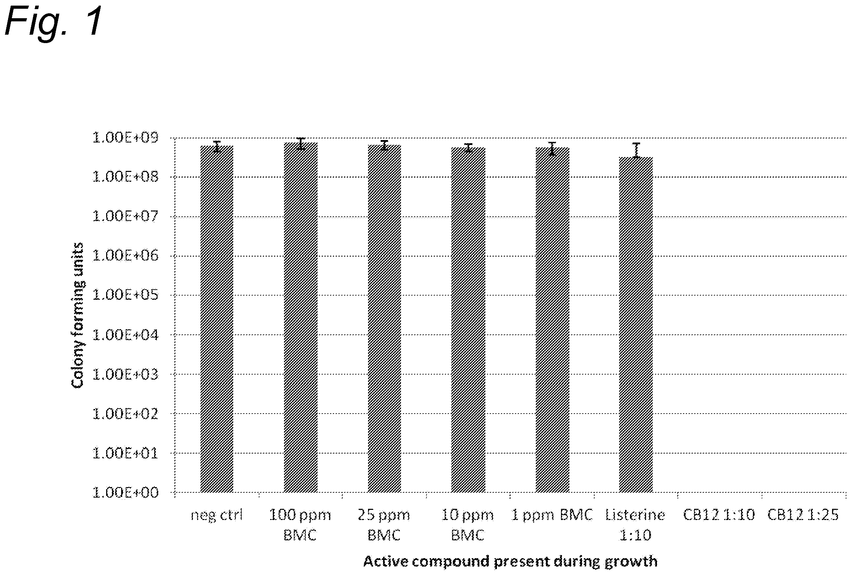

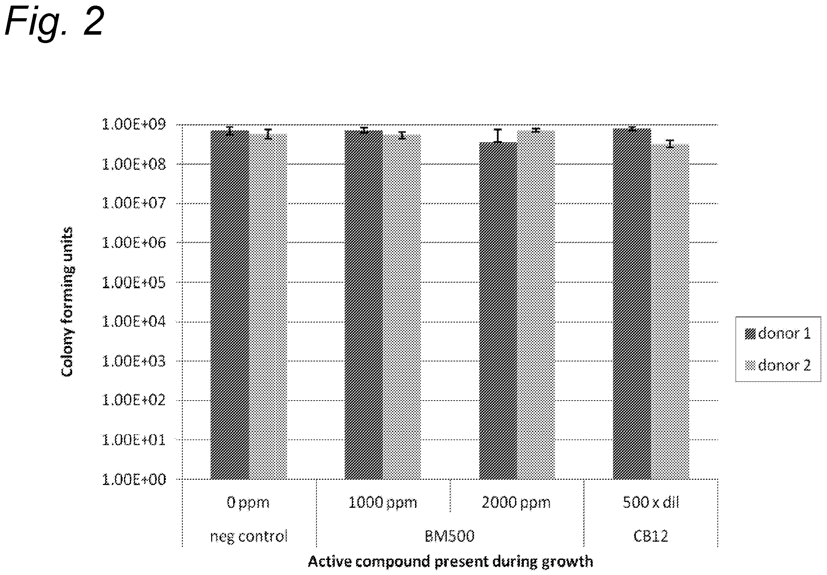

The data, as shown in fig. 1, show that BM500 does not affect the viability of 72hr grown microscosm biofilms at concentrations up to 100 ppm BM100 and even seems, although not statistically significant, to promote the growth of bacteria. The control products Listerine[R] and CB12 reduce the bacterial viability by respectively 50 and more than 99%. Figure 2 shows the effect of 1000 and 2000 ppm BM 500 and 500 times diluted CB12 (equivalent to 2000 ppm) on the viability of 72 hours microcosm biofilms of two different saliva donors; viability does not seem to be affected.

### Effect of BM500™ on microbial diversity and VSC production

To study the effect of the oral hygiene compositions according to the invention on volatile sulphur compound formation, bacterial halitosis biofilms were grown in glass-GC-vials over five days.

The GC-vials were inoculated with a saliva derived inoculum from four different donors. The media composition was similar to the bacterial viability assay.

The vials were closed airtight and were incubated at 37°C for 24 hours. After 24 hours the medium was refreshed with McBain medium containing either 1000, 2000 ppm BM500 or water as a negative control. The biofilms were allowed to grow under the same environmental conditions for an additional 96 hr, with a medium refreshment each 24 hours. We have varied the growth conditions by adding BM500™ in 1000 and 2000 ppm to the media.

After the growth period, volatile sulphur compound (VSC) production by the biofilms was scored organoleptically and by Oral Chroma measurement.

Prior to the Oral Chroma measurements, a 0.5 ml gas sample was taken from the GC vial and diluted with ambient air to quantifiable amounts.

After the determination of the gas composition, the biofilms were harvested and DNA was isolated. On the isolated DNA a PCR was performed using primers that will lift out a fragment of the bacterial 16S rDNA-gene. This gene is used for bacterial phylogeny.

16S rDNAfragments were separated and visualized using a Denaturing Gradient Gel Electrophoresis (DGGE) experiment. This DGGE analysis will give insight in the diversity of the samples; the treatment should not lead to biofilms with a low diversity, i.e. select for only specific species. DGGE will also give insight in similarities/differences between the bacterial community profiles of the samples; the hypothesis is that the treatment(s) will lead to a different bacterial community/different (nitrate reducing instead of VSC producing) species.

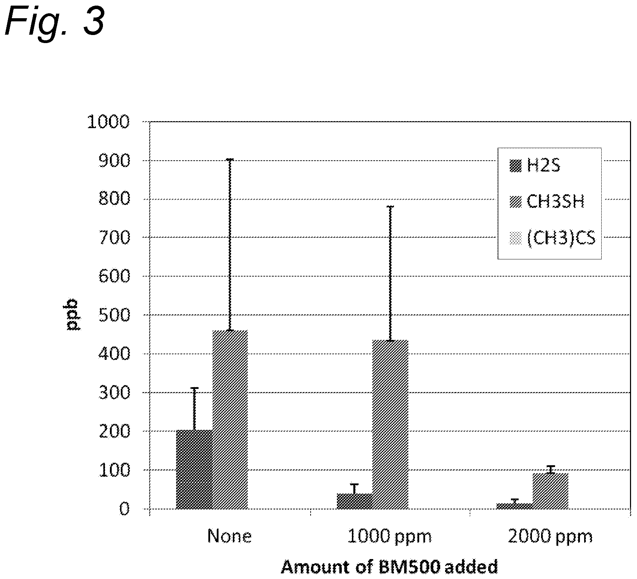

Moreover, the organoleptic scoring showed that biofilms treated with various concentrations of BM500 indeed produce markedly reduced VSC. The Oral Chroma measurements confirmed (figure 3) that the application of BM500 reduced the amount of $H_2S$. The amount of methyl mercaptane could not be determined using the Oral Chrome measurement. The amounts were below the detection limit of the Oral Chrome detection kit. Hence, the Oral Chrome measurement could neither confirm nor refute that methyl mercaptane was reduced. Reduction of methyl mercaptane should be confirmed using gas chromatography.

### *BM50O™ - treatment See bacterial viability assay*

Halitosis biofilms after the second medium refreshment were incubated in growth medium supplemented with different concentrations of BM500™. These concentrations BM500™ were subsequently kept in the growth medium until the end of the growth experiment.

#### *Read-outs*

*VSC production*

All biofilms were scored organoleptically on their VSC producing capacity.

*Denaturing Gradient Gel Electrophoresis*

After growth & organoleptic scoring the medium was removed from the tubes and replaced by 2mL of phosphate buffer saline (PBS) and vortexed and sonicated to remove the biofilms from the slides. Cells were subsequently pelleted and (selected) samples were processed for community profiling DGGE.

For DGGE bacterial DNA was extracted from selected samples using a Agowa kit (or equivalent). On this DNA a PCR was carried out using primer F357-GC, and primer R518 (Muyzer et al. Applied Environmental Microbiology 59, 695 (1993)).

The numbers in these primers reflect the positions in the bacterial 16s rDNA where they hybridize. Amplification was performed using Taq polymerase and a Biometra thermocycler (PCR machine) and yielded copies of the different bacterial 16S genes that were used for profiling.

DGGE was performed with the Bio-rad DCode system. The 16S PCR product was loaded onto 1-mm thick 8% (w/v) polyacrylamide (ratio of acrylamide to bisacrylamide, 37.5:1) gels containing a 30-70% linear denaturing gradient. To aid in the conversion and normalisation of the gels, a marker consisting of bacterial reference strains was added at both sides of each gel, as well as after every four samples. The gels were run at 40 V for 16 h, stained using ethidium bromide and recorded with a charge-coupled device camera system.

*Analysis of the community profiles*

Microbial diversity is the variation in microbial species in an ecosystem. To estimate microbial diversity, diversity indices are calculated statistically. A frequently used index is the *Shannon Weaver index of general diversity* (H'). H' takes into account the number of DGGE bands and the relative contribution of each band to the whole set of bands. Band positions will be assigned manually. The individual bands and their intensities in the community profiles will be determined using GelCompar software. A higher H' indicates a higher diversity.

Similarities (and hence also differences) between DGGE profiles will be calculated using GelCompar software by Pearson product-moment correlation coefficient and visualised using the Unweighted Pair Group Clustering Method with Arithmetic Averages (UPGMA). Analysis of the community profiles are performed as described in Pham LC, van Spanning RJ, Röling WF, Prosperi AC, Terefework Z, Ten Cate JM, Crielaard W, Zaura E. (2009) Effects of probiotic Lactobacillus salivarius W24 on the compositional stability of oralmicrobial communities. Arch Oral Biol Feb;54(2):132-7.

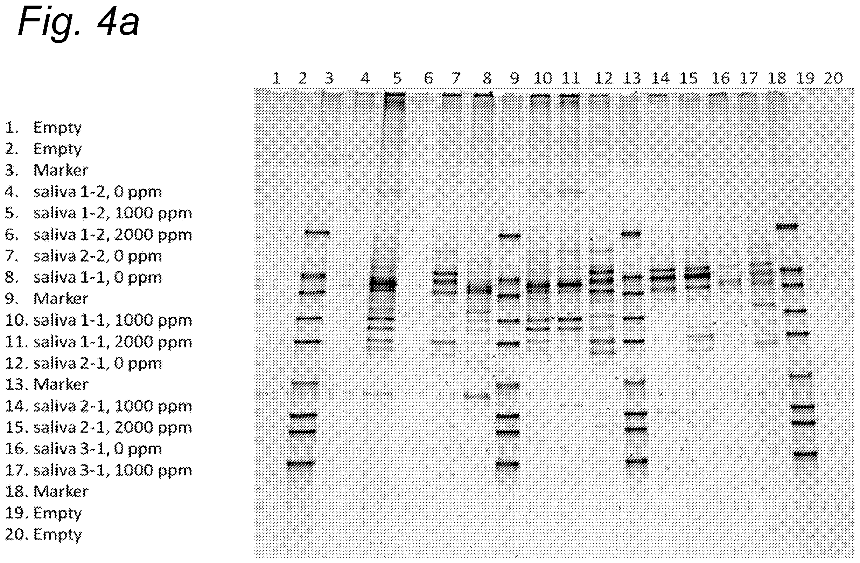

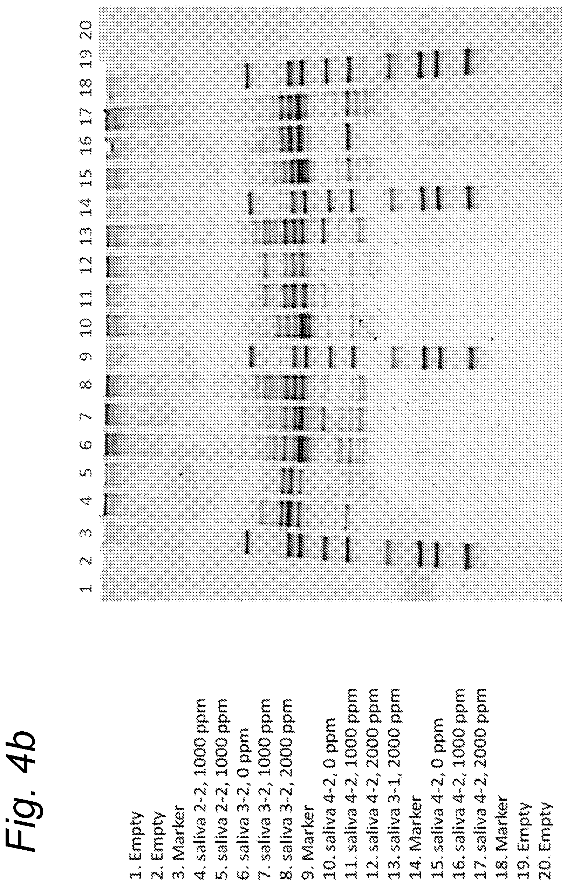

Figures 4a and 4b show the DGGE profiles from microcosm derived from four different saliva's (in duplo) grown under either 0, 1000 and 2000 ppm BM500. The samples show a shift in the microbial composition after treatment with 1000 or 2000 ppm BM500 as compared to the negative controls. For imaging purposes, black and white were inverted in Figures 4a and 4b.

It is clear, that the treatment with BM500 brings about a shift in the DGGE profiles, leading to the conclusion that a shift has taken place in the microbial ecosystem of the oral cavity. Determination of the Shannon-Weaver Index for untreated and treated populations, warranted the conclusion that the overall diversity of the microbial population is not diminished as a consequence of the treatment with BM500. On the contrary, treatment with BM500 gives rise to a shift towards anaerobic respirational growing (nitrate reducing) bacteria in a complex, healthy oral cavity ecology.

**EXAMPLE 2; The effect of BM500 rinse on oral microbiological changes and changes in microbiological activity related to halitosis in humans.**

### List of abbreviations and relevant definitions

| | |
|---|---|
| ABR | ABR form, General Assessment and Registration form, is the application form that is required for submission to the accredited Ethics Committee (In Dutch, ABR = Algemene Beoordeling en Registratie) |
| AE | Adverse Event |
| AR | Adverse Reaction |
| CA | Competent Authority |
| CCMO | Central Committee on Research Involving Human Subjects; in Dutch: Centrale Commissie Mensgebonden Onderzoek |
| CV | Curriculum Vitae |
| DSMB | Data Safety Monitoring Board |
| EU | European Union |
| EC | Ethics Commitee |
| EudraCT | European drug regulatory affairs Clinical Trials |
| GCP | Good Clinical Practice |
| IB | Investigator's Brochure |
| IC | Informed Consent |
| IMP | Investigational Medicinal Product |
| IMPD | Investigational Medicinal Product Dossier |

(continued)

| METC | Medical research ethics committee (MREC); in Dutch: medisch ethische toetsing commissie (METC) |
|------|------|
| NSG | Next Generation Sequencing |
| (S)AE | (Serious) Adverse Event |
| SPC | Summary of Product Characteristics (in Dutch: officiële productinfomatie IB1-tekst) |
| Sponsor | The sponsor is the party that commissions the organisation or performance of the research, for example a pharmaceutical company, academic hospital, scientific organisation or investigator. A party that provides funding for a study but does not commission it is not regarded as the sponsor, but referred to as a subsidising party. |
| SUSAR | Suspected Unexpected Serious Adverse Reaction |
| VAS | Visual Analogue Scale |
| VSCs | Volatile Sulphur Compounds |
| Wbp | Personal Data Protection Act (in Dutch: Wet Bescherming Persoonsgevens) |
| WMO | Medical Research Involving Human Subjects Act (in Dutch: Wet Medischwetenschappelijk Onderzoek met Mensen |

## SUMMARY

**Rationale:** An unbalanced oral microbial ecology can lead to several types of pathology and discomfort. These are usually tackled by the use of an anti-microbial mouthwash. These unspecific mouthwashes actually only reduce the total microbial load and do not re-establish microbial balance. In the current pilot trial an alternative approach will we evaluated where, instead of killing all microbes, specifically beneficial microbes will be stimulated. The imbalanced oral ecology that will be tackled is the one that leads to the discomfort of oral malodour (or the condition termed halitosis). Here strictly anaerobic microorganisms that via proteolytic activity produce (smelly) volatile sulphur compounds dominate oral microbial ecology. The hypothesis is that by using a mouth rinse that contains nitrate (BM500), conditions are changed in such a way that they favour nitrate-reducing microorganisms (firmly established as beneficial oral bacteria) and hence re-establishment of an ecological balance. As a result (i) proteolytic microorganisms will be outcompeted and (ii) production of smelly products will be reduced.

**Objective:** Determine if the use of BM500 oral rinse for 14 days has an effect on the composition of the oral microbiota. In addition, the effect on total microbial proteolytic activity and oral malodor will be determined.

**Study design:** This is a single centre, placebo controlled, double-blind exploratory pilot study in 2 groups of 10 systemically healthy volunteers.

**Study population:** 20 systemically healthy human volunteers, 18 - 55 yr old with halitosis as determined by organoleptic score.

**Intervention:** One group of 10 volunteers will rinse with BM500 (10 mL for 30 seconds) 3 times a day for a duration of 14 days and the other group of 10 volunteers will rinse with placebo (10 mL for 30 seconds) 3 times a day for a duration of 14 days.

**Main study parameters/endpoints:** Change in microbiota from baseline to day 14 and/or to day 31 as determined with 16S Next Generation Sequencing (NGS) microbiology.

**Nature and extent of the burden and risks associated with participation, benefit and group relatedness:** The risks for this study are negligible as the BM500 rinse contains components that are known to be non-hazardous/safe. In addition, the product will only be used to rinse very shortly and therefore it is anticipated that there will not be an actual uptake of any components.

The burden for the volunteers that participate in the study is minimal. They will make 6 short visits to the site and the data for this study will be obtained by non-invasive samples of the mouth (saliva, tongue and interproximal plaque). The benefit for the volunteers will be the possible relieve of halitosis discomfort.

## 1. INTRODUCTION AND RATIONALE

### 1.1 Background

Many pathologies and discomforts in the oral cavity are caused by a high abundance of microorganisms that do not dominate oral ecology under normal conditions. Caries is the result of an overload of acid producing bacteria, during gingivitis and periodontitis proteolytic bacteria dominate. These proteolytic bacteria, when overpopulating our tongue also lead to oral malodour (halitosis).

During pathology, e.g. periodontitis, current strategies usually involve the use of antimicrobial mouth rinses or even antibiotics. In the past years (Govoni et al 2008; Petersson et al 2009) it has become clear that these strategies have clear side effects. Antibiotics, beside side effects in the oral cavity (e.g. subsequent fungal infections), also lead to side effects on other (e.g. gut) human microbial ecology, which can even lead to diarrhoea. The unspecific antimicrobial mouth rinses also kill symbiotic bacteria in the oral cavity (Petersson et al 2009), a side effect, which until now has not received much attention, but underlines the need for alternative strategies. Certainly where microbial imbalance leads to discomfort, or a specific "condition", such as oral malodour (halitosis) drastic treatments should be avoided.

In the current trial we will aim to promote the growth of the symbiotic (Petersson et al 2009; Bryan 2012; Hezel & Weitzberg 2013; Hyde et al 2014) nitrate reducing bacteria on the tongue of volunteers. Specifically volunteers that have an overload of proteolytic bacteria, as indicated by oral malodour will be targeted. We will use a mouthrinse that contains nitrate (BM500). Preclinical work, where biofilms of oral bacteria were grown and treated in the laboratory has shown that indeed BM500, within 14 days, shifts microbial ecology, which leads to a less smelly bacterial composition

The volunteers will be asked to continue whatever oral hygiene measurements they are used to, but on top 3 times daily rinse with a BM500 solution for 2 weeks. The effect of this rinse regime on oral microbial ecology will be evaluated by using molecular techniques on 3 oral samples: saliva, plaque and tong coating. We will also evaluate how long the microbial shift will stay in place by repeating these analyses 2 weeks after stopping the rinsing regime. As an exploratory outcome we will also evaluate the reduction of proteolytic activity in saliva: bacteria responsible for oral malodour are those that produce proteases and hence can convert sulphur-containing proteins/amino acids into volatile (and smelly) sulphur confounds. Reduction in this activity will also be recorded organoleptically (smelling by trained operators) and via a simple gas chromatography (Oral Chroma).

### 1.2 Rationale

An unbalanced oral microbial ecology can lead to several types of pathology and discomfort. These are usually tackled by the use of an anti-microbial mouthwash. These unspecific mouthwashes actually only reduce the total microbial load and do not re-establish microbial balance. In the current pilot trial an alternative approach will we evaluate, where instead of killing all microbes, specifically beneficial microbes will be stimulated. The imbalanced oral ecology that will be tackled is the one that leads to the discomfort of oral malodour (or the condition termed halitosis). Here strictly anaerobic microorganisms that via proteolytic activity produce (smelly) volatile sulphur compounds dominate oral microbial ecology. The hypothesis is that by using a mouth rinse that contains nitrate (BM500), conditions are changed in such a way that they favour nitrate-reducing microorganisms (firmly established as beneficial oral bacteria) and hence re-establishment of an ecological balance. As a result (i) proteolytic microorganisms will be outcompeted and (ii) production of smelly products will be reduced.

### 2. OBJECTIVES

#### 2.1 Primary objective

This is an exploratory pilot study to determine if the use of BM500 oral rinse for 14 days has an effect on oral microbiological changes compared to placebo.

#### 2.2 Secundary objectives

In addition, the effect of the use of BM500 oral rinse for 14 days will be determined on the oral microbial activity related to halitosis such as proteolytic activity, the level of tongue coating and on oral malodour compared to placebo. Also, it will be determined how the BM500 oral rinse is experienced; the mouth feel (tolerance) will be examined using a VAS scale and compared with the placebo oral rinse.

### 3. STUDY DESIGN

This is an exploratory, pilot, single center, double-blind, placebo controlled study in 2 groups of 10 systemically healthy subjects with organoleptically determined halitosis. One group of 10 subjects will use the BM500 oral rinse and 10 subjects will use placebo mouth rinse, both for 14 consecutive days (rinsing 3 times a day).
Both groups will undergo the exact same assessments and will be monitored at baseline, just before the rinsing regime, after approximately 2 weeks rinsing and approximately 2 weeks after finishing rinsing. Overall, the subjects will visit the clinic 6 times over a period of approximately one month. For details of the visits and procedures see Table 1 under 7.3.4.

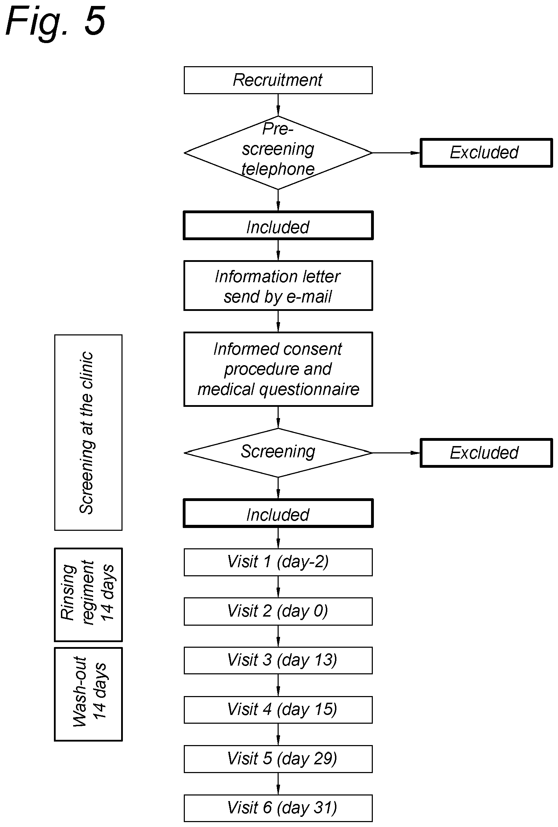

Figure 5 provides a flow chart of the procedures that the subjects will undergo in the course of the research.

## 4. STUDY POPULATION

### 4.1 Population

20 systemically healthy volunteers with halitosis (organoleptically determined) will be included in the study.

Initially, approximately 400 possible volunteers with self-reported halitosis will be recruited via adverts, websites and, or from existing databases. These subjects will be pre-screened (on general health, recent antibiotics use, recent dentist visits, medicine use, smoking) online or by phone.

Of these 400 subjects, 200 subjects will be screened in the clinic for halitosis (solely by organoleptic assessment). It is expected that of these 200 subjects, approximately 20% (i.e. 40 subjects) are expected score positive on halitosis (organoleptic score of $\geq$ 2) and can proceed with the actual screening for the study to determine if they meet all the specified in- and exclusion criteria:

### 4.2 Inclusion criteria

In order to be eligible to participate in this study, a subject must meet all of the following criteria:

- Age: $\geq$ 18 years - 55 year
- Male and female
- Classified as systemically healthy, assessed by medical questionnaire
- Organoleptic score of $\geq$ 2
- Presence of Volatile Sulphur Compounds (VSC's) using Oral Chroma®
- Minimum of 20 natural teeth: all first molars and second (or third molars) in the upper jaw available
- Agree to present with 'overnight' plaque
- Agree to present without eating and drinking 2 hours prior to visit
- Having visited the dentist for a regular check-up within the last year and having finished the necessary treatment.
- Willing and able to give written informed consent
- Willing to consent to use their collected anonymous and coded body materials for further research.

### 4.3 Exclusion criteria

A potential subject who meets any of the following criteria will be excluded from participation in this study:

- Anyone presenting with a probing depth $\geq$ 5mm with bleeding on probing and attachment loss $\geq$ 2 mm, Dutch Periodontal Screening Index score 3+/ 4
- Overt dental caries
- Interproximal restorations between all molars
- Smokers, definition non-smoker: <1 cigarette every day for at least one year
- Removable partial dentures
- Removable night guard
- Oral and/or peri-oral piercings
- Apparent oral lesions (aphthous ulcers excluded)
- Presence of orthodontic banding (except for lingual retention wire)
- Abuse of drugs/ alcohol
- ACTA dental student or ACTA professional
- Participation in a clinical study within the previous 30 days

General health and use of medication:

- Self-reported pregnancy or breastfeeding
- Use of antibiotics during the last 3 months
- Need of antibiotic prophylaxis prior to dental treatment
- Use of anti-inflammatory drugs on a regular basis
- Evidence of any systemic disease or compromised health condition
- Acute sinusitis
- Severe oral- pharyngeal infections
- Current disorders/disease resulting in (self-induced) vomiting
- Reduced salivary flow due to pathological reasons (e.g. Sjogren syndrome)

- Adverse medical history or long-term medication
- Prescribed medication (except for anti-contraceptives - birthcontrol pills)

**4.4 Sample size calculation**

It is anticipated that for screening, approximately 200 subjects are required to find a sufficient amount of subjects with halitosis. This is based on a study from Bornstein et al (2009) where 419 subjects were screened and 32% had an organoleptic score of $\geq 2$.

To calculate how many subjects we need to screen we use the following formula.

$$n >= \frac{N \times z^2 \times p(1-p)}{z^2 \times p(1-p) + (N-1) \times F^2}$$

n = number of subjects
z = standaarddeviation (1,96 at 95% confidence level)
N = population size (419)
p = chance of halitosis (32%)
F = error (5%)

$$186 >= \frac{419 \times 1,96^2 \times 32(1-32)}{1,96^2 \times 32(1-p32) + (419-1) \times 5^2}$$

For this exploratory pilot study we need a sample size of 2 groups of 10 subjects. The unpublished data of Exterkate et al is used to calculate the group size.

$$n = \left(\frac{Z\sigma}{E}\right)^2$$

((1.96*0.09)/0.05) = 13 subjects.

Taking into account a 40% drop-out at least 19 subjects are required. To get an even number for both test and placebo group; 10 subjects per group are needed.

It is expected that group size of n=10 for both the test product group and placebo group will be a sufficient number of subjects to be able to show an effect on the primary and exploratory outcomes in the test group compared to the placebo group.

**5. TREATMENT OF SUBJECTS**

The subjects will be randomly assigned to either the test group or the placebo group. The treatment frequency, duration and volume of oral rinsing is identical in both groups.

**5.1 Investigational product/treatment**

Test group: oral mouth rinse with BM500 3 times daily for a duration of 14 consecutive days. Each time the subject will rinse twice with 10 mL BM500 for 30 seconds (i.e. 14 days, 3x daily, 2x30 seconds with 2x10 mL).

Placebo group: oral mouth rinse with placebo mouthwash 3 times daily for a duration of 14 consecutive days. Each time the subject will rinse twice with 10 mL placebo for 30 seconds (i.e. 14 days, 3x daily, 2x30 seconds with 2x10 mL).

**6. INVESTIGATIONAL PRODUCT**

In this study BM500 will be used as a mouthwash/oral rinse. This oral rinse is considered a cosmetic product.

**6.1 Name and description of investigational product(s)**

The product that will be tested in this study is BM500 based mouthwash.

## 7. METHODS

### 7.1 Study parameters/endpoints

#### 7.1.1 Main study parameter/endpoint

The main study parameters are: Change in microbiota compared to baseline using 16S Next Generation Sequencing (NGS) microbiology for:

- saliva
- interproximal plaque
- tongue sample

#### 7.1.2 Secondary study parameters/endpoints

The secondary, exploratory outcome parameters are:

Reduction in total protease activity as determined by FRET from:

- saliva

Oral malodor by:

- organoleptic score

Change in Volatile Sulphur Compounds (VSCs) determined by OralChroma®

- breath sample

Extent of tongue coating and tongue body appearance:

- Discoloration
- Thickness
- Tongue body structure and shape

Tolerance in test group vs placebo group:

- Mouth feel using a VAS score

### 7.2 Randomisation, blinding and treatment allocation

The subjects will be randomly be assigned to either the test or the control treatment group.

### 7.3 Study procedures

#### 7.3.1 (Pre-)screening

Initially approximately 400 subjects will be pre-screened by phone or online. It will be determined if they are eligible for the study based on their general health, recent antibiotics use, recent dentist visits, medicine use, smoking habit and self-reported halitosis.

Approximately 200 of the pre-screened subjects are expected to be possible eligible after this pre-screening and will be invited to visit the clinic for an actual screening visit. Before the screening visit, these subjects will receive a written explanation of the background of the study, its objectives and involvement and they are asked to give their written informed consent. During the screening visit (visit 1), a dental hygienist performs an oral examination, including organoleptic scoring (for procedure and method see appendix 9.1). If this score is 2 or higher, the subjects will be screened on all in- and exclusion criteria.

Subjects complete a medical questionnaire in order to be classified as systemically healthy. The Dutch Periodontal Screening Index is scored.

The subjects that are screened and considered eligible to participate in the study will proceed with the baseline measurements for visit 1 (Day -2).

**7.3.2 Visit 1 (Day -2, baseline)**

During visit 1 (immediately following screening, i.e. on the same day) the following assessments are performed:

Stimulated fasting saliva sample

Two stimulated saliva samples will be collected (for microbiological analyses and proteolytic activity). For the procedure and method see appendix 9.2.

Interproximal plaque

Interproximal plaque will be collected with unwaxed dental floss between two upper molars. For the procedure and method see appendix 9.3.

Tongue sample (ecology)

The middle and posterior aspect of the dorsum of the tongue will be sampled with a microbrush. For the procedure and method see appendix 9.3.

Oral malodor

Determination of Volatile Sulphur Compounds (VSC's) using OralChroma®. For the procedure and method see appendix 9.4 and organoleptic score, already done as part of the screening (see 7.3.1).

Extent of tongue coating and tongue body appearance

Determination of discoloration, thickness and tongue body structure and shape. For the procedure and method see appendix 9.5.

**7.3.3 Visit 2**

Two days after the first visit, an additional baseline measurement will be performed Visit 2 (Day 0). During this the same assessments will be performed as described for Visit 1 (see also table below). In addition, the subjects will receive an explanation and instruction (on paper) on the use of oral rinse. Furthermore, the subjects will receive a diary to document the mouth rinse use and any adverse events. One day after this visit the subjects start using the BM500 mouth wash rinse or placebo for a period of 14 days. They are asked to start using the rinse from the morning of the next day (Day 1).

**7.3.4 Visit 3, 4, 5 and 6**

The subjects will visit the clinic another 4 times (i.e. once during the rinsing period and 3 times after the 14 day rinsing period): on Day 13, Day 15, Day 29 and Day 33. During these visits the same procedures as described for Visit 1 will be performed, including reporting of (S)AEs. Furthermore, during visit 4 and visit 5, the subjects are asked to describe their mouth feel using a VAS score. See Table 1 here below for details

**Table 1; Schedule of assessments**

| | Pre-screening | Screening and Visit 1 | Visit 2 | Visit 3 | | Visit 4 | Visit 5 | Visit 6 |
|---|---|---|---|---|---|---|---|---|
| | | Day -2 | Day 0 | Day 13 | Day 14 | Day 15 | Day 29 | Day 33 |
| Medical anamneses (update) | X | X | X | X | | X | X | X |
| Signing ICF | | X | | | | | | |

(continued)

|  | Pre-screening | Screening and Visit 1 | Visit 2 | Visit 3 |  | Visit 4 | Visit 5 | Visit 6 |
|---|---|---|---|---|---|---|---|---|
|  |  | Day -2 | Day 0 | Day 13 | Day 14 | Day 15 | Day 29 | Day 33 |
| Visit ACTA |  | X | X | X |  | X | X | X |
| In- and exclusion criteria |  | X |  |  |  |  |  |  |
| Hand-out rinse instructions and diary |  |  | X |  |  |  |  |  |
| Rinsing regimen BM500 or placebo |  |  | X# | X | X |  |  |  |
| Complete Diary |  |  | X# | X | X |  |  |  |
| Organoleptic score |  | X | X | X |  | X | X | X |
| Saliva sample (microbiological analysis + proteolytic activity) |  | X* | X | X |  | X | X | X |
| Interproximal plaque |  | X* | X | X |  | X | X | X |
| Tongue sample/plaque |  | X* | X | X |  | X | X | X |
| VSCs (oral malodor) |  | X* | X | X |  | X | X | X |
| Tongue coating and appearance |  | X* | X | X |  | X | X | X |
| (S)AE collection |  |  |  | X | X | X | X | X |
| VAS-score |  |  |  |  |  | X |  | X |
| *visit 1 only, # start Day 1 |  |  |  |  |  |  |  |  |

### 7.4 Withdrawal of individual subjects

Subjects can leave the study at any time for any reason if they wish to do so without any consequences. The investigator can decide to withdraw a subject from the study for urgent medical reasons.

### 7.5 Replacement of individual subjects after withdrawal

Subjects that withdraw from the study are not replaced.

### 7.6 Follow-up of subjects withdrawn from treatment

Follow-up of subjects withdrawn from treatment can be performed outside the research project in our regular clinics if subjects want to get help for oral malodour.

### 7.7 Premature termination of the study

In case of urgent medical reasons and/or unexpected severe or moderate adverse events in 4 subjects from the total population of 20 subjects, the study will be terminated prematurely.
Moderate adverse events are classified as symptoms causing enough discomfort to interfere with usual activity and severe incapacitating event causing inability to work or perform usual activity.

### 8. REFERENCES

- Bornstein MM, Kislig K, Hoti BB, Seemann R, Lussi A. Prevalence of halitosis in the population of the city of Bern, Switzerland: A study comparing self-reported and clinical data. Eur J Oral Sci 2009; 117:261-267.

- Bryan 2012; Bryan NS. (2012) Pharmacological therapies, lifestyle choices and nitric oxide deficiency: a perfect storm. Pharmacol Res. 66:448-56.

- Exterkate RAM, Zaura E, Brandt BW, Buijs MJ, Koopman JE, Crielaard W, ten Cate JM. Unpublished data. The effect of propidium monoazide treatment on the measured composition of clinical samples after the use of a mouthwash.

- Govoni et al 2008; Govoni M, Jansson EA, Weitzberg E, Lundberg JO. (2008) The increase in plasma nitrite after a dietary nitrate load is markedly attenuated by an antibacterial mouthwash. Nitric Oxide. 19:333-7.

- Hezel & Weitzberg 2013; Hezel M, Weitzberg E. (2013) The oral microbiome and nitric oxide homoeostasis. Oral Dis. 2013 Jun 28. doi: 10.1111/odi.12157.

- Hyde et al 2014;Hyde ER, Andrade F, Vaksman Z, Parthasarathy K, Jiang H, Parthasarathy DK, Torregrossa AC, Tribble G, Kaplan HB, Petrosino JF, Bryan NS. (2014) Metagenomic analysis of nitrate-reducing bacteria in the oral cavity: implications for nitric oxide homeostasis. PLoS One. 9:e88645.

- Larsen et al 2006; Larsen FJ, Ekblom B, Sahlin K, Lundberg JO, Weitzberg E. (2006) Effects of dietary nitrate on blood pressure in healthy volunteers. N Engl J Med. 355:2792-3.

- Petersson et al 2009; Petersson J, Carlström M, Schreiber O, Phillipson M, Christoffersson G, Jägare A, Roos S, Jansson EA, Persson AE, Lundberg JO, Holm L (2009) Gastroprotective and blood pressure lowering effects of dietary nitrate are abolished by an antiseptic mouthwash. Free Radic Biol Med. 46:1068-75.

## 9. APPENDICES

### 9.1 Oral malodour - Organoleptic score

**Procedure**

The organoleptic score will be determined by a trained and calibrated judges who is trained by Dr. Saad (Bristol). Inter and intra examiner reproducibility data are resulted as a moderate agreement. The judge tested his ability to distinguish odours using the Smell Identification Test® (Sensonics Inc., Haddon Heights, NJ, USA) and to detect odours at low concentrations, using a series of dilutions of the following substances: skatole, putrescine, isovaleric acid and dimethyl disulphide (Doty et al. 1984).

**Method**

Participants are instructed to close their mouth for 2 min, and then to slowly open their mouth at request of the examiner. Directly after opening the judge sniffs at a distance of approximately 3-5 cm from the nose of the examiner and the dorsum of the tongue of the subject. Subjects are instructed to breathe through the nose during the whole procedure. Two sniffers independent asses consecutive and subjective organoleptic measurements, using an arbitrary 0-5 scale (Rosenberg et al. 1991a, Rosenberg et al. 1991b further modified by Greenman et al. 2004). (The mean of both scores represented the individual organoleptic score).

Organoleptic score

0 = absence of odor
1 = barely noticeable odor
2 = slight odor
3 = moderate odor
4 = strong odor
5 = extremely strong odor

**References**

- Shaman P, Kimmelman CP, Dann MS. University of Pennsylvania Smell Identification Test: a rapid quantitative olfactory function test for the clinic. Laryngoscope. 1984 Feb;94(2 Pt 1):176-8.
- J Greenman J, Duffield J, Spencer P, Rosenberg M, Corry D, Saad S, Lenton P, Majerus G, Nachnani S, El-Maaytah M. Study on the organoleptic intensity scale for measuring oral malodor. J Dent Res. 2004 Jan;83(1):81-5.
- Rosenberg M, Kulkarni GV, Bosy A, McCulloch CA. Reproducibility and sensitivity of oral malodor measurements with a portable sulphide monitor. J Dent Res. 1991 Nov;70(11):1436-40.

**9.2 Stimulated saliva sample for microbiological analyses and proteolytic activity Procedure**

Stimulated saliva will be collected for microbiological analyses and proteolytic activity

**Method**

2x 5 ml saliva will be collected by spitting into a sterile vial while chewing on a piece of parafilm. During this process the vial will be cooled on ice. Saliva will be stored at -80°C until further processing.

**Microbial analysis**

Microbial DNA will be extracted (Kraneveld et al 2012). Samples will be processed for amplicon sequencing by barcoded masssequencing (Zaura et al, 2009). Data will be processed using dedicated bioinformatics pipeline installed at SARA (www.sara.nl). Microbial diversity analyses and phylogenetic profiles will be analyzed using both, OTU-based and phylogeny-based distance measures.

**Proteolytic analyses**

Saliva samples will be analyzed for protease activity using FRET technology.
This will be performed by following the enzymatic cleavage of fluorogenic BikKam substrates specifically tailored for oral proteolytic bacteria using a fluorescence microplate-reader Kaman et al, 2014). Protein concentration measurements (Bradford-assay; Bradford, 1972) will be performed on these samples for standardization/calibration.

**References:**

- Zaura E., Keijser B.J., Huse S.M., Crielaard W. (2009) Defining the healthy "core microbiome" of oral microbial communities. BMC Microbiol 9, 259-270
- Bradford, M.M. (1972). A rapid and sensitive method for the quantitation of microgram quantities of protein utilizing the principle of protein-dye binding. Anal Biochem 72: 248-254.
- Kraneveld EA, Buijs MJ, Bonder MJ, Visser M, Keijser BJF, Crielaard W, Zaura E (2012) The relation between oral Candida load and bacterial microbiome profiles in Dutch older adults. PLoS One. 2012;7(8):e42770.
- Kaman WE, Hays JPO, Endtz HO, Bikker FJ (2014) Bacterial proteases: targets for diagnostics and therapy. Eur J Clin Microbiol Infect Dis DOI 10.1007/s1 0096-014-2075-1

**9.3 Plaque and tongue samples for microbiological analyses**

**Procedure**

Interproximal plaque samples and tongue samples will be collected for microbiological analyses.

**Methods**

Interproximal plaque will be collected with unwaxed dental floss (Gerardu et al., 2007) between two upper molars. Mucosal swab samples will be collected from the tongue dorsum using a microbrush

**Interproximal plaque sample for microbiological profiling**

A sample will be taken of the unrestored interproximal site between the first and second molar or the second and the third molar in one randomly chosen quadrant of the upper jaw. Plaque will be collected with unwaxed dental floss and will be removed from the floss by drawing it through a slit cut in the lid (Gerardu et al., 2007) of a labeled Eppendorf vial with 50 $\mu$l RNAProtect (QIAGEN, Hilden, Germany). Immediately thereafter the plaque will be spun down using a microcentrifuge and stored on ice until transport to the laboratory and then stored at - 80°C until further processing for DNA extraction.

**Tongue sample for micobiological profiling**

Tongue mucosal swabs will be collected using a sterile microbrush (Microbrush International, Grafton, USA) by applying 4 strokes over the anterior two thirds of the tongue dorsum. Strokes will be made in an anterior-posterior direction.

After the sample is taken, the tip of the microbrush will be placed into an Eppendorf vial with 50 $\mu$l RNAProtect solution and clipped off. Samples will be stored at -80 °C till molecular analyses at the microbiology laboratory.

**Microbial analysis**

Microbial DNA will be extracted (Kraneveld et al 2012). Samples will be processed for amplicon sequencing by barcoded masssequencing (Zaura et al, 2009). Data will be processed using dedicated bioinformatics pipeline installed at SARA (www.sara.nl). Microbial diversity analyses and phylogenetic profiles will be analyzed using both, OTU-based and phylogeny-based distance measures.

**References:**

- Zaura E., Keijser B.J., Huse S.M., Crielaard W. (2009) Defining the healthy "core microbiome" of oral microbial communities. BMC Microbiol 9, 259-270
- Bradford, M.M. (1972). A rapid and sensitive method for the quantitation of microgram quantities of protein utilizing the principle of protein-dye binding. Anal Biochem 72: 248-254.
- Kraneveld EA, Buijs MJ, Bonder MJ, Visser M, Keijser BJF, Crielaard W, Zaura E (2012) The relation between oral Candida load and bacterial microbiome profiles in Dutch older adults. PLoS One. 2012;7(8):e42770.
- Kaman WE, Hays JPO, Endtz HO, Bikker FJ (2014) Bacterial proteases: targets for diagnostics and therapy. Eur J Clin Microbiol Infect Dis DOI 10.1007/s10096-014-2075-1
- Gerardu, V.A.M., Buijs, M.J., van Loveren, C. and ten Cate, J.M. (2007). Plaque formation and lactic acid production after the use of amine fluoride/stannous fluoride mouthrinse. EurJ Oral Sci 115: 148-152.

**9.4 Volatile Sulphur Compounds (VSC's) - Oral Chroma®**

**Procedure**

A portable gas chromatography (OralChroma®) using a flame photometric detector is the preferable method if precise measurements of specific gases are required. This technology is specifically designed to digitally measure molecular levels of the three major VSC ($H_2S$, $CH_3SH$, and dimethyl sulfide $CH_3SCH_3$). It is accurate in measuring the sulphur components of the breath and displays each gas concentration in graph form via computer interface.

**Method**

The volunteers are seated. The subjects are instructed to keep their mouth closed for 2 min and not to swallow, which allows sufficient build-up of VSC in the oral cavity. Upon the request of the examiner the mouth is opened, after which the mouth is slightly opened. Subjects should breath through the nose while the mouth is closed. A sterile disposable syringe is inserted through this opening into the oral cavity. The subject needs to avoid touching the tip of the syringe with the tongue. The piston is subsequently pulled to the very end of the syringe in order to fill the syringe with a breath sample from the oral cavity. The syringe is then removed from the oral cavity. After removing, saliva is wiped from the tip of the syringe with tissue paper and a gas injection needle is set onto the tip of the syringe. The piston of the syringe is slowly pushed to the 0.5 ml position. The remaining breath sample is injected into the OralChroma® in one stroke.

After 10 minutes the measurement is complete.

**OralChroma® readings**

The VSC reading of the OralChroma® shows the concentration values of hydrogen sulphide, methyl mercaptan and dimethyl sulphide in ppb and ng/ml. These values are recorded separately and chromatograms are printed for analysis.

**9.5 Extent of tongue coating**

**Digital Image**

Two digital images of the tongue surface are taken. One from the tip of the tongue and one from the posterior aspect.

- A digital camera Canon EOS D30 is used with Canon Macro Ring Lite MR-14EX and a CompactFlash Card FC-16M.
- The participant will take place in a dental chair in a upright position.
- Only the tongue will be included. Cheek retractors will be used.
- The images will taken centralized of the middle line of the tongue.

**Analyzing digital tongue images**

Tongue body color will be classified according the four typical tongue body colors (purple, pink, pale and red) (Yuen, 2002; Wang, David, 2001; Beaven, 1998).

**Procedure**

The procedure to assess tongue coating and appearance is a modification of the method as described by Miyazaki et al. (1995) and described in detail by Mantilla Gomez et al. (2001).

**Method**

Upon visual inspection the tongue is divided in 9 parts. From the vallate papillae to the tip i.e., back third, middle third and front third (according to Miyazaki et al. 1995). In addition from the left to the right i.e., left third, middle third and right third. For each of the 9 sections discoloration and coating is visually assessed.

**Tongue discoloration scale**

The discoloration is scored on a scale from 0 to 4:

0 =   0½ pink
1 =   1½ white
2 =   2½ yellow/ lightbrown
3 =   3½ brown
4 =   4½ black

**Tongue coating according to thickness**

The thickness is scored from a scale from 0 to 2:

0 =   0½ no coating,
1 =   1½ light-thin coating and
2 =   2½ heavy-thick coating

Light-thin-coating was scored when the pink color underneath is still visible through the coating. Heavy-thick coating is scored if no pink color can be observed under the coating. Each section of the tongue should be covered for more than 1/3 to obtain a score different than 0.

**EP 3 046 632 B1**

**References:**

- C.H. Li, P.C. Yuen. Tongue image matching using color content Pattern Recognition, 35 (2) (2002), pp. 407-419
- K.Q. Wang, Z. David, N.M. Li Tongue diagnosis based on biometric pattern recognition technology The World Scientific Publishers (2001) 575-98 p
- Donald Ward Beaven, Stafford Eric Brooks. A color atlas of the tongue in clinical diagnosis Publisher: Mosby, Incorporated Publication date: 2/1/1988 Pages: 256 ISBN-13: 9780815105879
- Gomez SM, Danser MM, Sipos PM, Rowshani B, Van der Velden U, Van der Weijden GA: Tongue coating and salivary bacterial counts in healthy/gingivitis subjects and periodontitis patients. J Clin Periodontol 2001; 28: 970-978. C Munksgaard, 2001
- Miyazaki H, Sakao S, Katoh Y, Takehara T. Correlation between volatile sulphur compounds and certain oral health measurements in the general population. J Periodontol. 1995 Aug; 66(8):679-84.

## Claims

1. An undiluted oral hygiene composition comprising 20 - 80 % nitrates of which at least half the amount of nitrates consists of calcium nitrate, the remainder of the nitrates being selected from any pharmaceutically acceptable cation salt thereof, 5 - 30% UHP (urea hydrogen peroxide), 0 - 20% urea, and water adding up to 100%.

2. An oral hygiene composition according to claim 1, comprising 30 - 75% nitrates.

3. An oral hygiene composition according to claim 1, comprising 40 - 70% nitrates.

4. An oral hygiene composition according to claim 1, comprising 50 - 65% nitrates.

5. An oral hygiene composition according to claim 1, wherein the other nitrates at least comprise ammonium nitrate and optionally potassium nitrate.

6. A diluted oral hygiene composition obtainable by diluting an undiluted oral hygiene composition according to any one of claims 1 to 5, wherein the dilution factor is selected from the group consisting of a between 1:1 and 1:10, between 1:10 and 1:50, between 1:50 and 1:100 and between 1:100 and 1: 1000.

7. An oral hygiene composition according to any one of claims 1 to 6 for use in the treatment and/or prevention of halitosis in a human or animal.

## Patentansprüche

1. Unverdünnte Mundpflegezusammensetzung umfassend 20-80% Nitrate, wobei wenigstens eine Hälfte der Menge der Nitrate aus Calciumnitrat besteht, wobei der Rest der Nitrate ausgewählt ist aus irgendeinem pharmazeutisch annehmbaren Kationensalz derselben, 5-30% UHP (Harnstoffwasserstoffperoxid), 0-20% Harnstoff und, auf 100% aufsummiert, Wasser.

2. Mundpflegezusammensetzung nach Anspruch 1, umfassend 30-75% Nitrate.

3. Mundpflegezusammensetzung nach Anspruch 1, umfassend 40-70% Nitrate.

4. Mundpflegezusammensetzung nach Anspruch 1, umfassend 50-65% Nitrate.

5. Mundpflegezusammensetzung nach Anspruch 1, wobei die anderen Nitrate wenigstens Ammoniumnitrat und optional Kaliumnitrat umfassen.

6. Verdünnte Mundpflegezusammensetzung, erhältlich durch Verdünnen einer unverdünnten Mundpflegezusammensetzung nach einem der Ansprüche 1 bis 5, wobei der Verdünnungsfaktor ausgewählt ist aus der Gruppe bestehend aus zwischen 1:1 und 1:10, zwischen 1:10 und 1:50, zwischen 1:50 und 1:100 und zwischen 1:100 und 1:1000.

7. Mundpflegezusammensetzung nach einem der Ansprüche 1 bis 6 zur Verwendung in der Behandlung und/oder Prävention von Mundgeruch bei einem Menschen oder Tier.

**Revendications**

1. Composition d'hygiène buccale non diluée comprenant 20 à 80 % de nitrates dont au moins la moitié de la quantité de nitrates est constituée de nitrate de calcium, le reste des nitrates étant choisi parmi tout sel de cation pharmaceutiquement acceptable de celui-ci, 5 à 30 % d'UHP (urée-peroxyde d'hydrogène), 0 à 20 % d'urée, et de l'eau s'ajoutant jusqu'à 100 %.

2. Composition d'hygiène buccale selon la revendication 1, comprenant 30 à 75 % de nitrates.

3. Composition d'hygiène buccale selon la revendication 1, comprenant 40 à 70 % de nitrates.

4. Composition d'hygiène buccale selon la revendication 1, comprenant 50 à 65 % de nitrates.

5. Composition d'hygiène buccale selon la revendication 1, dans laquelle les autres nitrates comprennent au moins du nitrate d'ammonium et éventuellement du nitrate de potassium.

6. Composition d'hygiène buccale diluée pouvant être obtenue par dilution d'une composition d'hygiène buccale non diluée selon l'une quelconque des revendications 1 à 5, dans lequel le facteur de dilution est choisi dans le groupe comprenant entre 1:1 et 1:10, entre 1:10 et 1:50, entre 1:50 et 1:100 et entre 1:100 et 1:1000.

7. Composition d'hygiène buccale selon l'une quelconque des revendications 1 à 6 pour le traitement et/ou la prévention de l'halitose chez un humain ou un animal.

Fig. 1

EP 3 046 632 B1

Fig. 2

*Fig. 3*

Fig. 4a

1. Empty
2. Empty
3. Marker
4. saliva 1-2, 0 ppm
5. saliva 1-2, 1000 ppm
6. saliva 1-2, 2000 ppm
7. saliva 2-2, 0 ppm
8. saliva 1-1, 0 ppm
9. Marker
10. saliva 1-1, 1000 ppm
11. saliva 1-1, 2000 ppm
12. saliva 2-1, 0 ppm
13. Marker
14. saliva 2-1, 1000 ppm
15. saliva 2-1, 2000 ppm
16. saliva 3-1, 0 ppm
17. saliva 3-1, 1000 ppm
18. Marker
19. Empty
20. Empty

# Fig. 4b

1. Empty
2. Empty
3. Marker
4. saliva 2-2, 1000 ppm
5. saliva 2-2, 1000 ppm
6. saliva 3-2, 0 ppm
7. saliva 3-2, 1000 ppm
8. saliva 3-2, 2000 ppm
9. Marker
10. saliva 4-2, 0 ppm
11. saliva 4-2, 1000 ppm
12. saliva 4-2, 2000 ppm
13. saliva 3-1, 2000 ppm
14. Marker
15. saliva 4-2, 0 ppm
16. saliva 4-2, 1000 ppm
17. saliva 4-2, 2000 ppm
18. Marker
19. Empty
20. Empty

# Fig. 5

## REFERENCES CITED IN THE DESCRIPTION

### Patent documents cited in the description

- WO 0174322 A1
- US 2008299051 A
- US 4525342 A
- US 4110429 A
- US 5145664 A
- US 5362737 A
- US 5374418 A
- US 2004067204 A
- US 2005239903 A
- WO 11094872 A1
- CN 102218021 A
- DE 102007050767 A1
- JP 8356310 A
- JP 98182384 A
- JP 63317621 A
- JP 90164816 A
- JP 60039538 A
- JP 86197510 A
- WO 9956714 A1
- GB 2381449 A
- CA 2154860
- US 2012148506 A
- KR 20030072766 A
- US 2006246015 A
- US 2009324547 A

### Non-patent literature cited in the description

- Effects of new flavonoid gums eliminating bad breath. Shokuhin Kogyo, 1995, vol. 38, 70-8
- **S. M. WALER.** The effect of zinc-containing chewing gum on volatile sulfur-containing compounds in the oral cavity. *Acta Odontol. Scand.,* 1997, vol. 55, 198-200
- **EXTERKATE et al.** *Caries Research,* 2010, vol. 44 (4), 372-379
- **MCBAIN et al.** *Journal of Applied Microbiology,* 2005, vol. 98, 624-634
- **MUYZER et al.** *Applied Environmental Microbiology,* 1993, vol. 59, 695
- **PHAM LC ; VAN SPANNING RJ ; RÖLING WF ; PROSPERI AC ; TEREFEWORK Z ; TEN CATE JM ; CRIELAARD W ; ZAURA E.** Effects of probiotic Lactobacillus salivarius W24 on the compositional stability of oralmicrobial communities. *Arch Oral Biol,* February 2009, vol. 54 (2), 132-7
- **BORNSTEIN MM ; KISLIG K ; HOTI BB ; SEEMANN R ; LUSSI A.** Prevalence of halitosis in the population of the city of Bern, Switzerland: A study comparing self-reported and clinical data. *Eur J Oral Sci,* 2009, vol. 117, 261-267
- **BRYAN NS.** Pharmacological therapies, lifestyle choices and nitric oxide deficiency: a perfect storm. *Pharmacol Res.,* 2012, vol. 66, 448-56
- **EXTERKATE RAM ; ZAURA E ; BRANDT BW ; BUIJS MJ ; KOOPMAN JE ; CRIELAARD W ; TEN CATE JM.** *Unpublished data. The effect of propidium monoazide treatment on the measured composition of clinical samples after the use of a mouthwash*
- **GOVONI M ; JANSSON EA ; WEITZBERG E ; LUNDBERG JO.** The increase in plasma nitrite after a dietary nitrate load is markedly attenuated by an antibacterial mouthwash. *Nitric Oxide.,* 2008, vol. 19, 333-7
- **HEZEL M ; WEITZBERG E.** The oral microbiome and nitric oxide homoeostasis. *Oral Dis.,* 28 June 2013
- **HYDE ER ; ANDRADE F ; VAKSMAN Z ; PARTHASARATHY K ; JIANG H ; PARTHASARATHY DK ; TORREGROSSA AC ; TRIBBLE G ; KAPLAN HB ; PETROSINO JF.** Metagenomic analysis of nitrate-reducing bacteria in the oral cavity: implications for nitric oxide homeostasis. *PLoS One,* 2014, vol. 9, e88645
- **LARSEN FJ ; EKBLOM B ; SAHLIN K ; LUNDBERG JO ; WEITZBERG E.** Effects of dietary nitrate on blood pressure in healthy volunteers. *N Engl J Med.,* 2006, vol. 355, 2792-3
- **PETERSSON J ; CARLSTRÖM M ; SCHREIBER O ; PHILLIPSON M ; CHRISTOFFERSSON G ; JÄGARE A ; ROOS S ; JANSSON EA ; PERSSON AE ; LUNDBERG JO.** Gastroprotective and blood pressure lowering effects of dietary nitrate are abolished by an antiseptic mouthwash. *Free Radic Biol Med.,* 2009, vol. 46, 1068-75
- **SHAMAN P ; KIMMELMAN CP ; DANN MS.** University of Pennsylvania Smell Identification Test: a rapid quantitative olfactory function test for the clinic. *Laryngoscope,* February 1984, vol. 94 (2), 176-8

- **J GREENMAN J ; DUFFIELD J ; SPENCER P ; ROSENBERG M ; CORRY D ; SAAD S ; LENTON P ; MAJERUS G ; NACHNANI S ; EL-MAAYTAH M.** Study on the organoleptic intensity scale for measuring oral malodor. *J Dent Res.,* January 2004, vol. 83 (1), 81-5
- **ROSENBERG M ; KULKARNI GV ; BOSY A ; MC-CULLOCH CA.** Reproducibility and sensitivity of oral malodor measurements with a portable sulphide monitor. *J Dent Res.,* November 1991, vol. 70 (11), 1436-40
- **ZAURA E. ; KEIJSER B.J. ; HUSE S.M. ; CRIE-LAARD W.** Defining the healthy ''core microbiome'' of oral microbial communities. *BMC Microbiol,* 2009, vol. 9, 259-270
- **BRADFORD, M.M.** A rapid and sensitive method for the quantitation of microgram quantities of protein utilizing the principle of protein-dye binding. *Anal Biochem,* 1972, vol. 72, 248-254
- **KRANEVELD EA ; BUIJS MJ ; BONDER MJ ; VISSER M ; KEIJSER BJF ; CRIELAARD W ; ZAURA E.** The relation between oral Candida load and bacterial microbiome profiles in Dutch older adults. *PLoS One,* 2012, vol. 7 (8), e42770
- **KAMAN WE ; HAYS JPO ; ENDTZ HO ; BIKKER FJ.** Bacterial proteases: targets for diagnostics and therapy. *Eur J Clin Microbiol Infect Dis,* 2014
- **GERARDU, V.A.M. ; BUIJS, M.J. ; VAN LOVEREN, C. ; TEN CATE, J.M.** Plaque formation and lactic acid production after the use of amine fluoride/stannous fluoride mouthrinse. *EurJ Oral Sci,* 2007, vol. 115, 148-152
- **C.H. LI ; P.C. YUEN.** *Tongue image matching using color content Pattern Recognition,* 2002, vol. 35 (2), 407-419
- **K.Q. WANG ; Z. DAVID ; N.M. LI.** Tongue diagnosis based on biometric pattern recognition technology The World. Scientific Publishers, 2001, 575-98
- **DONALD WARD BEAVEN ; STAFFORD ERIC BROOKS.** A color atlas of the tongue in clinical diagnosis. Mosby, Incorporated Publication, 02 January 1988, 256
- **GOMEZ SM ; DANSER MM ; SIPOS PM ; ROWSHANI B ; VAN DER VELDEN U ; VAN DER WEIJDEN GA.** Tongue coating and salivary bacterial counts in healthy/gingivitis subjects and periodontitis patients. *J Clin Periodontol,* 2001, vol. 28, 970-978
- **MIYAZAKI H ; SAKAO S ; KATOH Y ; TAKEHARA T.** Correlation between volatile sulphur compounds and certain oral health measurements in the general population. *J Periodontol.,* August 1995, vol. 66 (8), 679-84